# EUROPÄISCHE PATENTSCHRIFT

(11) **EP 3 433 351 B1**
(45) Veröffentlichungstag und Bekanntmachung des Hinweises auf die Patenterteilung: **14.01.2026**
(21) Anmeldenummer: 17712475.7
(22) Anmeldetag: 21.03.2017
(51) Int. Cl.: C11D 3/386

(54) **REINIGUNGSMITTEL ENTHALTEND PROTEASE UND AMYLASE**
CLEANING AGENT CONTAINING PROTEASE AND AMYLASE
PRODUIT DE NETTOYAGE CONTENANT DE LA PROTÉASE ET DE L'AMYLASE

(30) Priorität: 22.03.2016 DE 102016204061
(43) Veröffentlichungstag der Anmeldung: 30.01.2019
(73) Patentinhaber: Henkel AG & Co. KGaA, 40589 Düsseldorf (DE)
(72) Erfinder: BUISKER, Detlef, 45134 Essen (DE); SCHMELING, Marianne, 41352 Korschenbroich (DE); ZIPFEL, Johannes, 40593 Düsseldorf (DE); O'CONNELL, Timothy, 86899 Landsberg am Lech (DE)
(86) Internationale Anmeldenummer: PCT/EP2017/056656
(87) Internationale Veröffentlichungsnummer: WO 2017/162646

(56) Entgegenhaltungen:
- WO-A1-2013/087545
- WO-A1-2014/183920
- WO-A1-2015/149641
- YAN A. ET AL.: "GSP:BCF32192", 3 December 2015 (2015-12-03), XP055373517, Retrieved from the Internet <URL:http://ibis.internal.epo.org/exam/dbfetch.jsp?id=GSP:BCF32192> [retrieved on 20170516]
- YAN A. ET AL.: "GSP:BCF32197", 3 December 2015 (2015-12-03), XP055373515, Retrieved from the Internet <URL:http://ibis.internal.epo.org/exam/dbfetch.jsp?id=GSP:BCF32197> [retrieved on 20170516]
- MUSSMANN N. ET AL.: "EPOP:JC003961", 24 January 2014 (2014-01-24), XP055373272, Retrieved from the Internet <URL:http://ibis.internal.epo.org/exam/dbfetch.jsp?id=EPOP:JC003961> [retrieved on 20170516]
- ANDERSEN C. ET AL.: "GSP:BBQ08071", 1 January 2015 (2015-01-01), XP055371931, Retrieved from the Internet <URL:http://ibis.internal.epo.org/exam/dbfetch.jsp?id=GSP:BBQ08071> [retrieved on 20170511]
- TSUKAMOTO A ET AL: "Nucleotide sequence of the maltohexaose-producing amylase gene from an alkalophilic Bacillus sp. #707 and structural similarity to liquefying type @a-amylases", BIOCHEMICAL AND BIOPHYSICAL RESEARCH COMMUNICATIONS, ELSEVIER, AMSTERDAM, NL, vol. 151, no. 1, 29 February 1988 (1988-02-29), pages 25 - 31, XP024847498, ISSN: 0006-291X, [retrieved on 19880229], DOI: 10.1016/0006-291X(88)90554-2

## Beschreibung

Die vorliegende Anmeldung richtet sich auf ein Handgeschirrspülmittel, das mindestens eine Protease und mindestens eine Amylase enthält, wie hierin definiert, sowie die Verwendung eines solchen Mittels zum Reinigen von festen Oberflächen sowie ein Verfahren zur Reinigung von harten Oberflächen, unter Verwendung des beschriebenen Mittels. Schließlich betrifft die Erfindung auch die Verwendung einer speziellen Enzymkombination wie hierin definiert zur Verbesserung der Reinigungsleistung eines Handgeschirrspülmittels.

Das wichtigste Kriterium beim Reinigen von harten Oberflächen, wie insbesondere beim Geschirrspülen, ist die Reinigungsleistung an verschiedensten Anschmutzungen, welche insbesondere in Form von Lebensmittelresten eingebracht werden. Auch wenn die Reinigungsleistung der heutzutage eingesetzten Geschirrspülmittel generell hoch ist, stellt sich, auch aufgrund des generellen Trends beim Geschirrspülen vermehrt niedrige Temperaturen zu verwenden, allerdings das Problem, dass viele der üblichen Geschirrspülmittel bei hartnäckigen Anschmutzungen eine unzureichende Reinigungsleistung aufweisen. Eine solche unzureichende Reinigungsleistung und die damit einhergehende unzureichende Reinigung des Geschirrs führen beim Verbraucher zu Unzufriedenheit und dazu, dass solche Anschmutzungen vom Verbraucher vorbehandelt werden, was wiederum den Wasser- und Energieverbrauch erhöht. Daher besteht ein allgemeiner Bedarf nach Geschirrspülmitteln, die auch an hartnäckigen Anschmutzungen noch eine gute Reinigungsleistung aufweisen ohne dabei die bestehende gute Reinigungsleistung an anderen Anschmutzungen zu verringern.

Aus z.B. WO 2013/087545, WO 2014/183920 und WO 2015/149641 sind amylase- und proteasehaltige Wasch- und Reinigungsmittel bekannt.

Die Aufgabe der vorliegenden Erfindung bestand daher darin, ein Handgeschirrspülmittel, zur Verfügung zu stellen, das bei solchen Anschmutzungen eine gesteigerte Reinigungsleistung aufweist, ohne dass die Reinigungsleistung an anderen Anschmutzungen verringert wird.

Überraschenderweise wurde nun festgestellt, dass die Verwendung einer Kombination einer bestimmten Protease mit einer bestimmten Amylase die Reinigungsleistung von entsprechenden Handgeschirrspülmitteln an enzym-sensitiven Anschmutzungen in synergistischer Art und Weise verbessert.

In einem ersten Aspekt richtet sich die vorliegende Erfindung daher auf ein Handgeschirrspülmittel, das mindestens eine Protease und mindestens eine Amylase umfasst, wobei
a) die Amylase eine Aminosäuresequenz aufweist, die zu der in SEQ ID NO:1 angegebenen Aminosäuresequenz über deren Gesamtlänge zu 100% identisch ist; und
b) die mindestens eine Protease die Aminosäuresequenz gemäß SEQ ID NO:3 aufweist.

Eine solche Kombination bewirkt bei Anwendung des Mittels eine signifikant gesteigerte Reinigungsleistung an hartnäckigen Anschmutzungen, insbesondere an stärkehaltigen Anschmutzungen.

Ein weiterer Gegenstand der vorliegenden Erfindung ist die Verwendung eines hierin beschriebenen Handgeschirrspülmittels in einem Reinigungsverfahren, bevorzugt einem Geschirrspülverfahren, vorzugsweise die Verwendung zur Verbesserung der Reinigungsleistung, insbesondere der Reinigungsleistung an enzym-sensitiven Anschmutzungen, an harten Oberflächen, insbesondere Geschirr bei dessen Reinigung, insbesondere hartnäckigen Anschmutzungen, u.a. auch bei Temperaturen, die niedriger sind als die üblicherweise verwendeten Temperaturen.

Ein weiterer Gegenstand der vorliegenden Erfindung ist ein Geschirrspülverfahren, bei dem ein hierin beschriebenes Geschirrspülmittel, insbesondere zu dem Zweck, die Reinigungsleistung an enzym-sensitiven Anschmutzungen zu verbessern, zum Einsatz kommt. In verschiedenen Ausführungsformen der Erfindung werden bei dem Geschirrspülverfahren Temperaturen eingesetzt, die niedriger sind als die üblicherweise verwendeten Temperaturen.

Noch ein weiterer Gegenstand der Erfindung ist die Verwendung der hierin beschriebenen Enzymkombinationen zur Verbesserung der Reinigungsleistung eines Geschirrspülmittels.

"Niedrige Temperaturen" oder "Temperaturen, die niedriger sind als die üblicherweise verwendeten Temperaturen", wie hierin im Zusammenhang mit Geschirrspülverfahren verwendet, bezieht sich vorzugsweise auf Temperaturen unter 60 °C, insbesondere unter 55 °C, noch bevorzugter 50 °C oder niedriger, besonders bevorzugt 45 °C oder niedriger und am bevorzugtesten 40°C oder niedriger.

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist. Alle Prozentangaben sind, sofern nicht anders angegeben, Gewichts-%. Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

"Variante", wie hierin in Zusammenhang mit Enzymen verwendet, bezieht sich auf natürliche oder artifiziell erzeugte Variationen eines Enzyms, die eine gegenüber der Referenzform abgewandelte Aminosäuresequenz aufweisen. Eine solche Variante kann einzelne oder mehrere Punktmutationen, d.h. Substitutionen einer natürlicherweise an der entsprechenden Position vorkommenden Aminosäure durch eine andere, Insertionen (Einfügen von einer oder mehreren Aminosäuren) und/oder Deletionen (Entfernen von einer oder mehreren Aminosäuren) aufweisen, insbesondere eine oder mehrere Punktmutationen. Derartige Varianten haben vorzugsweise mindestens 50, vorzugsweise 60 oder mehr, noch bevorzugter 70, 80, 90, 100 % oder mehr der Enzymaktivität der Referenzform. In verschiedenen Ausführungsformen hat eine derartige Variante eine Aminosäuresequenz, die zu der als Referenz dienenden Sequenz über deren Gesamtlänge zu mindestens 70, vorzugsweise 75, 80, 85, 90, 95, 96, 97, 98, oder 99 % identisch ist. Die Varianten haben vorzugsweise dieselbe Länge wie die Referenzsequenz. Varianten können sich gegenüber der Referenzform durch verbesserte Eigenschaften auszeichnen, wie beispielsweise höhere Enzymaktivität, höhere Stabilität, geänderte Substratspezifität, etc.. Eingesetzt werden nur solche Varianten, die enzymatische Aktivität aufweisen. "Enzymatische Aktivität", wie in diesem Zusammenhang verwendet, bedeutet insbesondere, dass die entsprechenden Enzyme mindestens 50 %, vorzugsweise mindestens 90 % der katalytischen Aktivität ihres Referenzenzymes aufweisen.

"Fragment", wie hierin in Zusammenhang mit Enzymen verwendet, bezieht sich auf gegenüber dem Referenz-Enzym N-terminal und/oder C-terminal um jeweils eine oder mehrere Aminosäuren verkürzte Polypeptide. Eingesetzt werden nur solche Fragmente, die enzymatische Aktivität aufweisen. "Enzymatische Aktivität", wie in diesem Zusammenhang verwendet, bedeutet insbesondere, dass die entsprechenden Enzyme mindestens 50 %, vorzugsweise mindestens 90 % der katalytischen Aktivität ihres Referenzenzymes aufweisen.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Die eingesetzten Amylasen sind alkalische a-Amylasen. Sie wirken als Hydrolasen und spalten die α(1-4)-Glykosidbindung von Polysacchariden, insbesondere Stärken wie Amylose, und bewirken dadurch den Abbau stärkehaltiger Anschmutzungen auf dem Reinigungsgut. Als Spaltprodukte entstehen dabei Dextrine und daraus Maltose, Glucose und verzweigte Oligosaccharide. Ihr pH-Optimum liegt meist im deutlich alkalischen Bereich.

Als Amylase im Sinne der vorliegenden Erfindung wird eine Amylase eingesetzt, die eine Aminosäuresequenz umfasst, die zu der in SEQ ID NO:1 angegebenen Aminosäuresequenz über deren Gesamtlänge zu 100% identisch ist.

Die hierin beschriebenen Mittel enthalten ferner mindestens eine Protease. Die Proteasen sind insbesondere alkalische Serin-Proteasen. Sie wirken als unspezifische Endopeptidasen, das heißt, sie hydrolysieren beliebige Säureamidbindungen, die im Inneren von Peptiden oder Proteinen liegen und bewirken dadurch den Abbau proteinhaltiger Anschmutzungen auf dem Reinigungsgut. Ihr pH-Optimum liegt meist im deutlich alkalischen Bereich.

Die mindestens eine Protease umfasst eine alkalische Protease aus *Bacillus lentus* DSM 5483 oder ein funktionales Fragment oder eine Variante davon. Die Sequenz der reifen (maturen) alkalische Protease aus *Bacillus lentus* DSM 5483 ist in SEQ ID NO:2 angegeben. Eine derartige Protease weist eine Aminosäuresequenz auf, die in der Zählung gemäß SEQ ID NO:2 die Aminosäuresubstitutionen R99E, S3T, V4I und V199I aufweist. Eine derartige Protease ist in SEQ ID NO:3 angegeben.

In verschiedenen Ausführungsformen werden diese Kombinationen von Amylase und Protease im Massenverhältnis bezogen auf Aktivprotein von 10:1 bis 1:10, vorzugsweise 5:1 bis 1:5, besonders bevorzugt von 3:1 bis 1:3, noch bevorzugter 1:1 bis 1:3, am meisten bevorzugt 1:2 eingesetzt.

In verschiedenen Ausführungsformen ist die mindestens eine Amylase in dem erfindungsgemäßen Mittel in Mengen von 0,01 bis 2,0 Gew.-%, vorzugsweise 0,05 bis 1,5 Gew.-%, noch bevorzugter 0,1 bis 1,0 Gew.-% bezogen auf Aktivprotein und das Gesamtgewicht des Mittels enthalten.

In verschiedenen Ausführungsformen ist die mindestens eine Protease in dem erfindungsgemäßen Mittel in Mengen von 0,01 bis 2,0 Gew.-%, vorzugsweise 0,05 bis 1,5 Gew.-%, am bevorzugtesten 0,1 bis 1,0 Gew.-% bezogen auf Aktivprotein und das Gesamtgewicht des Mittels enthalten.

Überraschenderweise zeigen die hierin beschriebenen Kombinationen von bestimmten Amylasen und Proteasen die Eigenschaft, die Leistung des Handgeschirrspülmittels zu verbessern, indem sie zu einer synergistisch verbesserten Reinigungsleistung an hartnäckigen Anschmutzungen führen.

Dabei ist in der Regel unter der Verbesserung der Reinigungsleistung zu verstehen, dass bei Verwendung der hierin beschriebenen Mittel die Entfernung von Anschmutzungen, auf harten Oberflächen, insbesondere Geschirr, bei dessen Reinigung im Vergleich zu der Verwendung von Mitteln, die die hierin beschriebenen Enzymkombinationen nicht enthalten, merklich verbessert ist.

Die einzusetzenden Enzyme können ferner zusammen mit Begleitstoffen, etwa aus der Fermentation, oder mit Stabilisatoren konfektioniert sein. In flüssigen Formulierungen werden die Enzyme bevorzugt als Enzymflüssigformulierung(en) eingesetzt.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren bestimmt werden. Die Bestimmung der Aktivproteinkonzentration erfolgt diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al., J. Am. Chem. Soc. 88, 24 (1966), S. 5890-5913).

Enzyme, insbesondere die hierin beschriebenen Amylasen und Proteasen, können besonders während der Lagerung gegen Schädigungen wie beispielsweise Inaktivierung, Denaturierung oder Zerfall etwa durch physikalische Einflüsse, Oxidation oder proteolytische Spaltung geschützt werden.

Bei mikrobieller Gewinnung ist eine Inhibierung der Proteolyse besonders bevorzugt. Die beschriebenen Mittel können zu diesem Zweck Stabilisatoren enthalten.

Reinigungsaktive Enzyme werden in der Regel nicht in Form des reinen Proteins, sondern vielmehr in Form stabilisierter, lager- und transportfähiger Zubereitungen bereitgestellt. Zu diesen vorkonfektionierten Zubereitungen zählen beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalienundurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

Wie aus der vorherigen Ausführungen ersichtlich, bildet das Enzym-Protein nur einen Bruchteil des Gesamtgewichts üblicher Enzym-Zubereitungen. Bevorzugt eingesetzte Enzym-Zubereitungen enthalten zwischen 0,1 und 40 Gew.-%, bevorzugt zwischen 0,2 und 30 Gew.-%, besonders bevorzugt zwischen 0,4 und 20 Gew.-% und insbesondere zwischen 0,8 und 15 Gew.-% des Enzymproteins. Die beschriebenen Mittel enthalten derartige Enzym-Zubereitungen daher vorzugsweise in einer Menge so dass die oben angegeben Aktivproteinkonzentrationen erreicht werden.

Die hierin beschriebenen Mittel können fester oder flüssiger Natur sein und insbesondere als homogene Lösungen oder Suspensionen vorliegen.

Die hierin beschriebenen Mittel können Tenside enthalten, wobei zur Gruppe der Tenside die nichtionischen, die anionischen, die kationischen und die amphoteren Tenside gezählt werden.

Als nichtionische Tenside können alle dem Fachmann bekannten nichtionischen Tenside eingesetzt werden. Als nichtionische Tenside eignen sich beispielsweise Alkylglykoside der allgemeinen Formel RO(G)ₓ, in der R einem primären geradkettigen oder methylverzweigten, insbesondere in 2-Stellung methylverzweigten aliphatischen Rest mit 8 bis 22, vorzugsweise 12 bis 18 C-Atomen entspricht und G das Symbol ist, das für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.

Eine weitere Klasse bevorzugt einsetzbarer nichtionischer Tenside, die entweder als alleiniges nichtionisches Tensid oder in Kombination mit anderen nichtionischen Tensiden eingesetzt werden können, sind alkoxylierte, vorzugsweise ethoxylierte oder ethoxylierte und propoxylierte Fettsäurealkylester, vorzugsweise mit 1 bis 4 Kohlenstoffatomen in der Alkylkette.

Weitere geeignete Tenside sind die als PHFA bekannten Polyhydroxyfettsäureamide.

Als bevorzugte Tenside können schwachschäumende nichtionische Tenside eingesetzt werden. Mit besonderem Vorzug enthalten die Mittel nichtionische Tenside aus der Gruppe der alkoxylierten Alkohole. Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 Mol EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt einer ganzen oder einer gebrochenen Zahl entsprechen können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

In verschiedenen bevorzugten Ausführungsformen enthalten die Mittel der Erfindung als nichtionisches Tensid mindestens ein Aminoxid. Prinzipiell sind diesbezüglich alle im Stand der Technik für diese Zwecke etablierten Aminoxide, also Verbindungen, die die Formel R¹R²R³NO aufweisen, worin jedes R¹, R² und R³ unabhängig von den anderen eine gegebenenfalls substituierte, beispielsweise Hydroxy-substituierte, C₁-C₃₀ Kohlenwasserstoffkette ist, einsetzbar. Besonders bevorzugt eingesetzte Aminoxide sind solche in denen R¹ C₁₂-C₁₈ Alkyl und R² und R³ jeweils unabhängig C₁-C₄ Alkyl sind, insbesondere C₁₂-C₁₈ Alkyldimethylaminoxide. Beispielhafte Vertreter geeigneter Aminoxide sind N-Kokosalkyl-N,N-dimethylaminoxid und N-Talgalkyl-N,N-dihydroxyethylaminoxid. C₁₂-C₁₈ Alkyldimethylaminoxide bzw. Mischungen, die auch C₁₆ und C₁₈-Alkyldimethylaminoxide enthalten, sind gegenüber C₁₂-C₁₄ Alkyldimethylaminoxiden bevorzugt. In gleicher Art und Weise sind generell Aminoxide in denen R¹ auch C₁₆- und C₁₈-Reste umfasst gegenüber solchen, in denen R¹ nur C₁₂₋₁₄ Reste umfasst, bevorzugt. In verschiedenen Ausführungsformen enthalten die eingesetzten Aminoxide daher mindestens 20 Gew.-%, vorzugsweise mindestens 30 Gew.-%, noch bevorzugter mindestens 40 Gew.-%, am bevorzugtesten mindestens 50 Gew.-%, 60 Gew.-%, 70 Gew.-% oder 80 Gew.-% C₁₆₋₁₈ Aminoxide bezogen auf das Gesamtgewicht der Aminoxide. In solchen Ausführungsformen kann der Rest der Aminoxide aus C₁₂₋₁₄ Aminoxiden bestehen. Das mindestens eine Aminoxid wird vorzugsweise in einer Menge von 0,5 bis 10,0 Gew.-%, noch bevorzugter 0,8 bis 8,0 Gew.-% bezogen auf das Gesamtgewicht des Mittels eingesetzt.

In verschiedenen bevorzugten Ausführungsformen wird auch mindestens ein Aniontensid eingesetzt. Zu ihnen zählen insbesondere Alkylbenzolsulfonate, (Fett-)Alkylsulfate, (Fett-)Alkylethersulfate sowie Alkansulfonate. Die Mittel der Erfindung enthalten vorzugsweise mindestens ein anionisches Tensid aus der Gruppe der Alkylethersulfate. Bevorzugte Alkylethersulfate sind solche der Formel (I)

R¹-O-(AO)ₙ-SO₃⁻ X⁺ (I).

In dieser Formel (I) steht R¹ für einen linearen oder verzweigten, substituierten oder unsubstituierten Alkylrest, vorzugsweise für einen linearen, unsubstituierten Alkylrest, besonders bevorzugt für einen Fettalkoholrest. Bevorzugte Reste R¹ sind ausgewählt aus Decyl-, Undecyl-, Dodecyl-, Tridecyl-, Tetradecyl, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R¹ sind abgeleitet von C₁₂-C₁₈-Fettalkoholen, beispielsweise von Kokosfettalkohol, Talgfettalkohol, Lauryl-, Myristyl-, Cetyl- oder Stearylalkohol oder von C₁₀-C₂₀-Oxoalkoholen. X steht für ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen X⁺ können ausgewählt sein aus NH₄⁺, ½ Zn²⁺,½ Mg²⁺,½ Ca²⁺,½ Mn²⁺, und deren Mischungen.

AO steht für eine Ethylenoxid- (EO) oder Propylenoxid- (PO) Gruppierung, vorzugsweise für eine Ethylenoxidgruppierung. Der Index n steht für eine ganze Zahl von 1 bis 50, vorzugsweise von 1 bis 20 und insbesondere von 2 bis 10. Ganz besonders bevorzugt steht n für die Zahlen 2, 3, 4, 5, 6, 7 oder 8.

Bevorzugt sind Fettalkoholethersulfate der Formel I-1 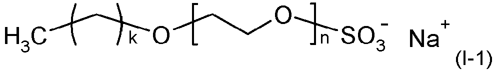 mit k = 11 bis 19, n = 2, 3, 4, 5, 6, 7 oder 8. Besonders bevorzugte Vertreter sind Na-C₁₂₋₁₄ Fettalkoholethersulfate mit 2 EO (k = 11-13, n = 2 in Formel II-1).

Die Alkylethersulfate werden vorzugsweise in einer Menge von 3,0 bis 30,0 Gew.-%, noch bevorzugter 4,0 bis 25,0 Gew.-%, am bevorzugtesten 4,0 bis 18,0 Gew.-% bezogen auf das Gesamtgewicht des Mittels eingesetzt.

Schließlich enthalten die Mittel der Erfindung in verschiedenen bevorzugten Ausführungsformen weiterhin mindestens ein Betain. Geeignete Betaine sind solche der Formel (Rⁱⁱⁱ)(R^{iv})(R^{v})N⁺CH₂COO⁻, in der Rⁱⁱⁱ einen gegebenenfalls durch Heteroatome oder Heteroatomgruppen, wie beispielsweise O, S, NH, C(O), C(O)NH oder C(O)O, unterbrochenen Alkylrest mit 8 bis 25, vorzugsweise 10 bis 21 Kohlenstoffatomen und R^{iv} sowie R^{v} gleichartige oder verschiedene Alkylreste mit 1 bis 3 Kohlenstoffatomen bedeuten. Besonders bevorzugt sind C₁₀-C₁₈-Alkyl-dimethylcarboxymethylbetain und C₁₁-C₁₇-Alkylamidopropyl-dimethylcarboxymethylbetain. Das mindestens eine Betain wird vorzugsweise in einer Menge von 0,9 bis 15,0 Gew.-%, noch bevorzugter 1,0 bis 12,0 Gew.-%, am bevorzugtesten 1,5 bis 8,0 Gew.-% bezogen auf das Gesamtgewicht des Mittels eingesetzt.

Darüber hinaus können in den Mitteln der Erfindung zusätzlich zu den oben genannten weitere Tensid eingesetzt werden. Geeignet sind beispielsweise Tenside aus den Gruppen der Alkylbenzolsulfonate, Alkylsulfate, Alkylestersulfonate, sekundären Alkansulfonate, Fettalkoholalkoxylate, Alkylglykoside, alkoxylierte Fettsäurealkylester, Fettsäurealkanolamide, Hydroxymischether, Sorbitanfettsäurester, Polyhydroxyfettsäureamide und alkoxylierte Alkohole. Derartige Tenside sind im Stand der Technik bekannt.

In verschiedenen Ausführungsformen beträgt die Gesamtmenge der Tenside bezogen auf das Gewicht des Mittels 4 bis 45 Gew.-%, vorzugsweise 6 bis 40 Gew.-%, noch bevorzugter 10 bis 32 Gew.-%.

In einer Ausführungsform sind die Mittel gemäß der vorliegenden Erfindung flüssig und enthalten Wasser als Hauptlösungsmittel, d.h. es handelt sich um wässrige Mittel. Der Wassergehalt des erfindungsgemäßen wässrigen Mittels beträgt üblicherweise 15 bis 95 Gew.-%, vorzugsweise 20 bis 90 Gew.-%. In verschiedenen Ausführungsformen beträgt der Wassergehalt mehr als 5 Gew.-%, bevorzugt mehr als 15 Gew.-% und insbesondere bevorzugt mehr als 25 Gew.-%, jeweils bezogen auf die Gesamtmenge an Mittel.

Daneben können dem Mittel nichtwässrige Lösungsmittel zugesetzt werden. Geeignete nichtwässrige Lösungsmittel umfassen ein- oder mehrwertige Alkohole, Alkanolamine oder Glykolether, sofern sie im angegebenen Konzentrationsbereich mit Wasser mischbar sind. Vorzugsweise werden die Lösungsmittel ausgewählt aus Ethanol, n-Propanol, i-Propanol, Butanolen, Glykol, Propandiol, Butandiol, Methylpropandiol, Glycerin, Diglykol, Propyldiglycol, Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethylether, Propylenglykolethylether, Propylenglykolpropylether, Dipropylenglykolmonomethylether, Dipropylenglykolmonoethylether, Methoxytriglykol, Ethoxytriglykol, Butoxytriglykol, 1-Butoxyethoxy-2-propanol, 3-Methyl-3-methoxybutanol, Propylenglykol-t-butylether, Di-n-octylether sowie Mischungen dieser Lösungsmittel.

Das eine oder die mehreren nichtwässrigen Lösungsmittel ist/sind üblicherweise in einer Menge von 0,1 bis 10 Gew.-%, vorzugsweise 1 bis 8 Gew.-% bezogen auf die Gesamtzusammensetzung enthalten.

Neben den bisher genannten Komponenten können die erfindungsgemäßen Mittel weitere Inhaltsstoffe enthalten, die die anwendungstechnischen und/oder ästhetischen Eigenschaften des Mittels weiter verbessern. Hierzu zählen beispielsweise Additive zur Verbesserung des Ablauf- und Trocknungsverhaltens, zur Einstellung der Viskosität und/oder zur Stabilisierung, sowie weitere in Mitteln übliche Hilfs- und Zusatzstoffe, etwa UV-Stabilisatoren, Parfüm, Perlglanzmittel, Farbstoffe, Korrosionsinhibitoren, Konservierungsmittel, Bitterstoffe, organische Salze, Desinfektionsmittel, strukturgebende Polymere, Entschäumer, verkapselte Inhaltsstoffe (z.B. verkapseltes Parfum), pH-Stellmittel sowie Hautgefühl-verbessernde oder pflegende Additive.

Zur weiteren Verbesserung des Ablauf- und/oder Trocknungsverhaltens kann das erfindungsgemäße Mittel ein oder mehrere Additive aus der Gruppe der Polymere und der Buildersubstanzen (Builder) enthalten, üblicherweise in einer Menge von 0,001 bis 5 Gew.-%, vorzugsweise 0,01 bis 4 Gew.-%, insbesondere 0,1 bis 3 Gew.-%, besonders bevorzugt 0,2 bis 2 Gew.-%, äußerst bevorzugt 0,5 bis 1,5 Gew.-%, beispielsweise 1 Gew.-%.

Polymere Verdickungsmittel im Sinne der vorliegenden Erfindung sind die als Polyelektrolyte verdickend wirkenden Polycarboxylate, vorzugsweise Homo- und Copolymerisate der Acrylsäure, insbesondere Acrylsäure-Copolymere wie Acrylsäure-Methacrylsäure-Copolymere, und die Polysaccharide, insbesondere Heteropolysaccharide, sowie andere übliche verdickende Polymere.

Geeignete Polysaccharide bzw. Heteropolysaccharide sind die Polysaccharidgummen, beispielsweise Gummi arabicum, Agar, Alginate, Carrageene und ihre Salze, Guar, Guaran, Tragacant, Gellan, Ramsan, Dextran oder Xanthan und ihre Derivate, z.B. propoxyliertes Guar, sowie ihre Mischungen. Andere Polysaccharidverdicker, wie Stärken oder Cellulosederivate, können alternativ, vorzugsweise aber zusätzlich zu einem Polysaccharidgummi eingesetzt werden, beispielsweise Stärken verschiedensten Ursprungs und Stärkederivate, z.B. Hydroxyethylstärke, Stärkephosphatester oder Stärkeacetate, oder Carboxymethylcellulose bzw. ihr Natriumsalz, Methyl-, Ethyl-, Hydroxyethyl-, Hydroxypropyl-, Hydroxypropyl-methyl- oder Hydroxyethyl-methyl-cellulose oder Celluloseacetat.

Als polymere Verdickungsmittel geeignete Acrylsäure-Polymere sind beispielsweise hochmolekulare mit einem Polyalkenylpolyether, insbesondere einem Allylether von Saccharose, Pentaerythrit oder Propylen, vernetzte Homopolymere der Acrylsäure (INCI Carbomer), die auch als Carboxyvinylpolymere bezeichnet werden.

Besonders geeignete polymere Verdickungsmittel sind aber folgende Acrylsäure-Copolymere: (i) Copolymere von zwei oder mehr Monomeren aus der Gruppe der Acrylsäure, Methacrylsäure und ihrer einfachen, vorzugsweise mit C₁-₄-Alkanolen gebildeten, Ester (INCI Acrylates Copolymer), zu denen etwa die Copolymere von Methacrylsäure, Butylacrylat und Methylmethacrylat (CAS 25035-69-2) oder von Butylacrylat und Methylmethacrylat (CAS 25852-37-3) gehören; (ii) vernetzte hochmolekulare Acrylsäurecopolymere, zu denen etwa die mit einem Allylether der Saccharose oder des Pentaerythrits vernetzten Copolymere von C₁₀-₃₀-Alkylacrylaten mit einem oder mehreren Monomeren aus der Gruppe der Acrylsäure, Methacrylsäure und ihrer einfachen, vorzugsweise mit C₁-₄-Alkanolen gebildeten, Ester (INCI Acrylates/C10-30 Alkyl Acrylate Crosspolymer) gehören.

Der Gehalt an polymerem Verdickungsmittel beträgt üblicherweise nicht mehr als 8 Gew.-%, vorzugsweise zwischen 0,1 und 7 Gew.-%, besonders bevorzugt zwischen 0,5 und 6 Gew.-%, insbesondere zwischen 1 und 5 Gew.-% und äußerst bevorzugt zwischen 1,5 und 4 Gew.-%, beispielsweise zwischen 2 und 2,5 Gew.-%, bezogen auf das Gesamtgewicht des Mittels.

Zur Stabilisierung des erfindungsgemäßen Mittels, insbesondere bei hohem Tensidgehalt, können ein oder mehrere Dicarbonsäuren und/oder deren Salze zugesetzt werden, insbesondere eine Zusammensetzung aus Na-Salzen der Adipin-, Bernstein- und Glutarsäure, wie sie z.B. unter dem Handelsnamen Sokalan^{®} DSC erhältlich ist. Der Einsatz erfolgt hierbei vorteilhafterweise in Mengen von 0,1 bis 8 Gew.-%, vorzugsweise 0,5 bis 7 Gew.-%, insbesondere 1,3 bis 6 Gew.-% und besonders bevorzugt 2 bis 4 Gew.-%, bezogen auf das Gesamtgewicht des Mittels.

Kann jedoch auf deren Einsatz verzichtet werden, so ist das erfindungsgemäße Mittel vorzugsweise frei von Dicarbonsäure(salze)n.

Daneben können noch ein oder mehrere weitere - insbesondere in Handgeschirrspülmitteln - übliche Hilfs- und Zusatzstoffe, insbesondere UV-Stabilisatoren, Parfüm, Perlglanzmittel (INCI Opacifying Agents; beispielsweise Glykoldistearat, z.B. Cutina^{®} AGS der Fa. Cognis, bzw. dieses enthaltende Mischungen, z.B. die Euperlane^{®} der Fa. Cognis), Farbstoffe, Korrosionsinhibitoren, Konservierungsmittel (z.B. das technische auch als Bronopol bezeichnete 2-Brom-2-nitropropan-1,3-diol (CAS 52-51-7), das beispielsweise als Myacide^{®} BT oder als Boots Bronopol BT von der Firma Boots gewerblich erhältlich ist), organische Salze, Desinfektionsmittel, Enzyme, pH-Stellmittel sowie Hautgefühl-verbessernde oder hautpflegende Additive (z.B. dermatologisch wirksame Substanzen wie Vitamin A, Vitamin B2, Vitamin B12, Vitamin C, Vitamin E, D-Panthenol, Sericerin, Collagen-Partial-Hydrolysat, verschiedene pflanzliche Protein-Partial-Hydrolysate, Proteinhydrolysat-Fettsäure-Kondensate, Liposome, Cholesterin, pflanzliche und tierische Öle wie z.B. Lecithin, Sojaöl, usw., Pflanzenextrakte wie z.B. Aloe Vera, Azulen, Hamamelisextrakte, Algenextrakte, usw., Allantoin, A.H.A.-Komplexe, Glycerin, Harnstoff, quaternisierte Hydroxyethylcellulose), in Mengen von üblicherweise nicht mehr als 5 Gew.-% enthalten sein, bezogen auf das Gesamtgewicht des Mittels.

Der pH-Wert des erfindungsgemäßen Mittel kann mittels üblicher pH-Regulatoren, beispielsweise Säuren wie Mineralsäuren oder Citronensäure und/oder Alkalien wie Natrium- oder Kaliumhydroxid, eingestellt werden, wobei - insbesondere bei gewünschter Handverträglichkeit - ein Bereich von 4 bis 9, vorzugsweise 5 bis 8, insbesondere 5,5 bis 7,5, bevorzugt ist.

Zur Einstellung und/oder Stabilisierung des pH-Werts kann das erfindungsgemäße Mittel ein oder mehrere Puffer-Substanzen (INCI Buffering Agents) enthalten, üblicherweise in Mengen von 0,001 bis 5 Gew.-%, vorzugsweise 0,005 bis 3 Gew.-%, insbesondere 0,01 bis 2 Gew.-%, besonders bevorzugt 0,05 bis 1 Gew.-%, äußerst bevorzugt 0,1 bis 0,5 Gew.-%, beispielsweise 0,2 Gew.-%. Bevorzugt sind Puffer-Substanzen, die zugleich Komplexbildner oder sogar Chelatbildner (Chelatoren, INCI Chelating Agents) sind. Besonders bevorzugte Puffer-Substanzen sind die Citronensäure bzw. die Citrate, insbesondere die Natrium- und Kaliumcitrate, beispielsweise Trinatriumcitrat·2 H₂O und Trikaliumcitrat·H₂O.

Das erfindungsgemäße Mittel lässt sich zur manuellen Reinigung von Geschirr verwenden.

Die Herstellung des Mittels erfolgt mittels üblicher und bekannter Methoden und Verfahren.

Gegenstand der vorliegenden Erfindung ist schließlich auch ein Verfahren zur Reinigung von harten Oberflächen, das dadurch gekennzeichnet ist, dass in mindestens einem Verfahrensschritt ein hierin beschriebenes Mittel angewendet wird.

Ein weiterer Erfindungsgegenstand ist die Verwendung eines hierin beschriebenen Mittels zur Reinigung von harten Oberflächen.

Noch ein weiterer Erfindungsgegenstand ist die Verwendung der hierin beschriebenen Enzymkombinationen zur Verbesserung der Spülleistung eines Handgeschirrspülmittels.

Alle Sachverhalte, Gegenstände und Ausführungsformen, die für hierin beschriebene Mittel beschrieben sind, sind auch auf die vorstehend genannten Verfahren und Verwendungen anwendbar. Daher wird an dieser Stelle ausdrücklich auf die Offenbarung an entsprechender Stelle verwiesen mit dem Hinweis, dass diese Offenbarung auch für die vorstehenden beschriebenen Verfahren und Verwendungen gilt.

### Beispiele

### Beispiel 1: Formulierungen

Erfindungsgemäße Rezeptur (E1)
4,5 % FAEOS
1,5 % Betain
1,0 % Aminoxid
0,1 % Amylase (SEQ ID NO:1)
0,4 % Protease (SEQ ID NO:3)
ad 100 % Parfüm, Farbe, Konservierungsmittel, Wasser

Referenz-Rezeptur 1 (V1)
4,5 % FAEOS
1,5 % Betain
1,0 % Aminoxid
0,21 % Amylase (Termamyl 300L (Novozymes, DK))
0,39 % Protease (Alcalase^{®} (Novozymes, DK))
ad 100 % Parfüm, Farbe, Konservierungsmittel, Wasser

Referenz-Rezeptur 2 (V2)
4,5 % FAEOS
1,5 % Betain
1,0 % Aminoxid
0,21 % Amylase (Thermamyl 300L)
0,43 % Protease (Biotouch^{®} ROC 250 L (AB Enzymes, DE))
ad 100 % Parfüm, Farbe, Konservierungsmittel, Wasser

Referenz-Rezeptur 3 (V3)
4,5 % FAEOS
1,5 % Betain
1,0 % Aminoxid
0,21 % Amylase (Thermamyl 300L)
0,34 % Protease (Purafect^{®} Prime 4000 L (DuPont, US))
ad 100 % Parfüm, Farbe, Konservierungsmittel, Wasser

Referenz-Rezeptur 4 (V4)
4,5 % FAEOS
1,5 % Betain
1,0 % Aminoxid
0,1 % Amylase (SEQ ID NO:1)
0,43 % Protease (Biotouch^{®} ROC 250 L (AB Enzymes, DE))
ad 100 % Parfüm, Farbe, Konservierungsmittel, Wasser

Referenz-Rezeptur 5 (V5)
4,5 % FAEOS
1,5 % Betain
1,0 % Aminoxid
0,21 % Amylase (Termamyl 300L)
0,4 % Protease (SEQ ID NO:3)
ad 100 % Parfüm, Farbe, Konservierungsmittel, Wasser

Referenz-Rezeptur 5 (V5)
4,5 % FAEOS
1,5 % Betain
1,0 % Aminoxid
0,21 % Amylase (Termamyl 300L)
0,4 % Protease (SEQ ID NO:3)
ad 100 % Parfüm, Farbe, Konservierungsmittel, Wasser

Referenz-Rezeptur 6 (V6)
19,1 % FAEOS
7,2 % Betain
4,8 % Aminoxid
0,3 % Amylase (SEQ ID NO:1)
ad 100 % Parfüm, Farbe, Konservierungsmittel, Wasser

Referenz-Rezeptur 7 (V7)
19,1 % FAEOS
7,2 % Betain
4,8 % Aminoxid
0,6 % Amylase (SEQ ID NO:1)
ad 100 % Parfüm, Farbe, Konservierungsmittel, Wasser

Referenz-Rezeptur 8 (V8)
19,1 % FAEOS
7,2 % Betain
4,8 % Aminoxid
0,3 % Protease (SEQ ID NO:3)
ad 100 % Parfüm, Farbe, Konservierungsmittel, Wasser

Referenz-Rezeptur 9 (V9)
19,1 % FAEOS
7,2 % Betain
4,8 % Aminoxid
0,6 % Protease (SEQ ID NO:3)
ad 100 % Parfüm, Farbe, Konservierungsmittel, Wasser

Erfindungsgemäße Rezeptur 2 (E2)
19,1 % FAEOS
7,2 % Betain
4,8 % Aminoxid
0,3 % Amylase (SEQ ID NO:1)
0,3 % Protease (SEQ ID NO:3)
ad 100 % Parfüm, Farbe, Konservierungsmittel, Wasser

Alle Angaben sind Gew.-% Aktivsubstanz bezogen auf das Gesamtgewicht.

### Beispiel 2: Reinigungsleistung

| | **Durchschnittliche Stärkeentfernung in % (Stärkeanschmutzung)** | **Durchschnittliche Proteinentfernung in % (Käseanschmutzung*)** |
|---|---|---|
| E1 | 92 | 92 |
| V1 | 20 | 85 |
| V2 | 16 | 76 |
| V3 | 22 | 75 |
| V4 | 70 | 86 |
| V5 | 21 | 64 |
| V6 | | 57 |
| V7 | | 61 |
| V8 | | 37 |
| V9 | | 40 |
| E2 | | 77 |

| | | |
|---|---|---|
| *Bei der Käseanschmutzung handelt es sich um eine handelsübliche ''4 cheese sauce", wie sie von verschiedenen Anbietern erhältlich ist, die auf Teller bzw. Stahlbleche aufgebracht und eingebrannt wird. | | |

Ersichtlich wird, dass die erfindungsgemäße Enzymkombination auf stärke- und protein-haltigen Anschmutzungen wirksamer ist als die jeweiligen Enzyme allein bzw. Kombinationen anderer im Stand der Technik bekannter Enzyme.

## Patentansprüche

1. Handgeschirrspülmittel, **dadurch gekennzeichnet, dass** das Mittel mindestens eine Protease und mindestens eine Amylase umfasst, wobei
a) die Amylase eine Aminosäuresequenz aufweist, die zu der in SEQ ID NO:1 angegebenen Aminosäuresequenz über deren Gesamtlänge zu 100% identisch ist; und
b) die mindestens eine Protease die Aminosäuresequenz gemäß SEQ ID NO:3 aufweist.

2. Das Mittel gemäß Anspruch 1, **dadurch gekennzeichnet, dass**
(i) die mindestens eine Amylase und die mindestens eine Protease im Massenverhältnis bezogen auf Aktivprotein von 10:1 bis 1:10, vorzugsweise 5:1 bis 1:5, besonders bevorzugt von 3:1 bis 1:3, noch bevorzugter 1:1 bis 1:3, am meisten bevorzugt 1:2 eingesetzt werden; und/oder
(ii) die mindestens eine Amylase in Mengen von 0,01 bis 2,0 Gew.-%, vorzugsweise 0,05 bis 1,5 Gew.-% bezogen auf Aktivprotein und das Gesamtgewicht des Mittels eingesetzt wird; und/oder
(iii) die mindestens eine Protease in Mengen von 0,01 bis 2,0 Gew.-%, vorzugsweise 0,05 bis 1,5 Gew.-% bezogen auf Aktivprotein und das Gesamtgewicht des Mittels eingesetzt wird.

3. Das Mittel gemäß einem der vorhergehenden Ansprüche, **dadurch gekennzeichnet, dass** das Mittel ferner Tenside enthält, wobei die Gesamtmenge der Tenside bezogen auf das Gewicht des Mittels 4 bis 45 Gew.-%, vorzugsweise 6 bis 40 Gew.-%, noch bevorzugter 10 bis 32 Gew.-% beträgt.

4. Das Mittel gemäß einem der vorhergehenden Ansprüche, **dadurch gekennzeichnet, dass** das Mittel
(i) mindestens ein Aminoxid, vorzugsweise ein C₁₂-C₁₈ Alkyldimethylaminoxid, vorzugsweise in einer Menge von 0,5 bis 10,0 Gew.-%, noch bevorzugter 0,8 bis 8,0 Gew.-% bezogen auf das Gesamtgewicht des Mittels;
(ii) mindestens ein Ethersulfat, vorzugsweise in einer Menge von 3,0 bis 30,0 Gew.-%, noch bevorzugter 4,0 bis 25,0 Gew.-% bezogen auf das Gesamtgewicht des Mittels; und
(iii) mindestens ein Betain, vorzugsweise in einer Menge von 0,9 bis 15,0 Gew.-%, noch bevorzugter 1,0 bis 12,0 Gew.-% bezogen auf das Gesamtgewicht des Mittels enthält.

5. Das Mittel gemäß einem der vorhergehenden Ansprüche, **dadurch gekennzeichnet, dass** das Mittel mindestens einen zusätzlichen Inhaltsstoff ausgewählt aus der Gruppe bestehend aus Additiven zur Verbesserung des Ablauf- und Trocknungsverhaltens, zur Einstellung der Viskosität und/oder zur Stabilisierung, UV-Stabilisatoren, Parfüm, Perlglanzmitteln, Farbstoffen, Korrosionsinhibitoren, Konservierungsmitteln, Bitterstoffen, organischen Salzen, Desinfektionsmitteln, strukturgebenden Polymeren, Entschäumern, verkapselten Inhaltsstoffen, pH-Stellmitteln sowie Hautgefühl-verbessernden oder pflegenden Additiven enthält.

6. Verwendung eines Handgeschirrspülmittels nach einem der Ansprüche 1 bis 5 zum Reinigen von festen Oberflächen.

7. Verfahren zur Reinigung von harten Oberflächen, **dadurch gekennzeichnet, dass** in mindestens einem Verfahrensschritt ein Handgeschirrspülmittel nach einem der Ansprüche 1 bis 5 angewendet wird.

8. Verwendung einer Enzymkombination umfassend mindestens eine Protease und mindestens eine Amylase, wobei
a) die Amylase eine Aminosäuresequenz aufweist, die zu der in SEQ ID NO:1 angegebenen Aminosäuresequenz über deren Gesamtlänge zu 100% identisch ist; und
b) die mindestens eine Protease die Aminosäuresequenz gemäß SEQ ID NO:3 aufweist, zur Verbesserung der Spülleistung eines Handgeschirrspülmittels.

## Claims

1. Hand dishwashing detergent, **characterized in that** the detergent comprises at least one protease and at least one amylase, wherein
a) the amylase has an amino acid sequence that is 100% identical to the amino acid sequence specified in SEQ ID NO:1 over its entire length; and
b) the at least one protease has the amino acid sequence according to SEQ ID NO:3.

2. The detergent according to claim 1, **characterized in that**
(i) the at least one amylase and the at least one protease are used in a mass ratio based on active protein of 10:1 to 1:10, preferably 5:1 to 1:5, more preferably 3:1 to 1:3, even more preferably 1:1 to 1:3, most preferably 1:2; and/or
(ii) the at least one amylase is used in amounts of 0.01 to 2.0 wt.%, preferably 0.05 to 1.5 wt.%, based on active protein and the total weight of the agent; and/or
(iii) the at least one protease is used in amounts of 0.01 to 2.0% by weight, preferably 0.05 to 1.5% by weight, based on active protein and the total weight of the agent.

3. The agent according to one of the preceding claims, **characterized in that** the agent further contains surfactants, wherein the total amount of surfactants, based on the weight of the agent, is 4 to 45 wt.%, preferably 6 to 40 wt.%, more preferably 10 to 32 wt.%.

4. The agent according to one of the preceding claims, **characterized in that** the agent contains
(i) at least one amine oxide, preferably a C₁₂-C₁₈ alkyldimethylamine oxide, preferably in an amount of 0.5 to 10.0 wt.%, more preferably 0.8 to 8.0 wt.% based on the total weight of the agent;
(ii) at least one ether sulphate, preferably in an amount of 3.0 to 30.0% by weight, more preferably 4.0 to 25.0% by weight, based on the total weight of the agent; and
(iii) at least one betaine, preferably in an amount of 0.9 to 15.0% by weight, more preferably 1.0 to 12.0% by weight, based on the total weight of the agent.

5. The agent according to one of the preceding claims, **characterized in that** the agent contains at least one additional ingredient selected from the group consisting of additives for improving the draining and drying behaviour, for adjusting the viscosity and/or for stabilisation, UV stabilisers, perfume, pearlescent agents, dyes, corrosion inhibitors, preservatives, bittering agents, organic salts, disinfectants, structuring polymers, defoamers, encapsulated ingredients, pH adjusters and skin feel-improving or skin care additives.

6. Use of a hand dishwashing detergent according to any of claims 1 to 5 for cleaning solid surfaces.

7. Method for cleaning hard surfaces, **characterized in that** a hand dishwashing detergent according to one of claims 1 to 5 is used in at least one step of the method.

8. Use of an enzyme combination comprising at least one protease and at least one amylase, wherein
a) the amylase has an amino acid sequence that is 100% identical to the amino acid sequence specified in SEQ ID NO:1 over its entire length; and
b) the at least one protease has the amino acid sequence according to SEQ ID NO:3,
for improving the rinsing performance of a hand dishwashing detergent.

## Revendications

1. Produit vaisselle à usage domestique, **caractérisé en ce qu'**il comprend au moins une protéase et au moins une amylase, dans lequel
a) l'amylase présente une séquence d'acides aminés qui est identique à 100 % à la séquence d'acides aminés indiquée dans SEQ ID NO:1 sur toute sa longueur ; et
b) la au moins une protéase présente la séquence d'acides aminés selon SEQ ID NO:3.

2. Le produit selon la revendication 1, **caractérisé en ce que**
(i) la au moins une amylase et la au moins une protéase sont utilisées dans un rapport massique, par rapport à la protéine active, de 10:1 à 1:10, de préférence de 5:1 à 1:5, de manière particulièrement préférée de 3:1 à 1:3, de manière encore plus préférée de 1:1 à 1:3, et de manière la plus préférée de 1:2 ; et/ou
(ii) la au moins une amylase est utilisée en quantités de 0,01 à 2,0 % en poids, de préférence de 0,05 à 1,5 % en poids par rapport à la protéine active et au poids total de l'agent ; et/ou
(iii) la au moins une protéase est utilisée en quantités de 0,01 à 2,0 % en poids, de préférence de 0,05 à 1,5 % en poids, par rapport à la protéine active et au poids total de l'agent.

3. L'agent selon l'une des revendications précédentes, **caractérisé en ce que** l'agent contient en outre des tensioactifs, la quantité totale des tensioactifs par rapport au poids de l'agent étant comprise entre 4 et 45 % en poids, de préférence entre 6 et 40 % en poids, et plus préférentiellement encore entre 10 et 32 % en poids.

4. Le produit selon l'une des revendications précédentes, **caractérisé en ce que** le produit
(i) au moins un oxyde d'amine, de préférence un oxyde d'alkyldiméthylamine en C₁₂ à C₁₈, de préférence en une quantité de 0,5 à 10,0 % en poids, de préférence encore de 0,8 à 8,0 % en poids par rapport au poids total de l'agent ;
(ii) au moins un sulfate d'éther, de préférence en une quantité de 3,0 à 30,0 % en poids, de préférence encore de 4,0 à 25,0 % en poids par rapport au poids total de l'agent ; et
(iii) au moins une bétaïne, de préférence en une quantité comprise entre 0,9 et 15,0 % en poids, de préférence encore entre 1,0 et 12,0 % en poids par rapport au poids total du produit.

5. Le produit selon l'une des revendications précédentes, **caractérisé en ce que** le produit contient au moins un ingrédient supplémentaire choisi dans le groupe constitué par les additifs destinés à améliorer le comportement au ruissellement et au séchage, à ajuster la viscosité et/ou à stabiliser, les stabilisateurs UV, parfums, agents nacrants, colorants, inhibiteurs de corrosion, conservateurs, substances amères, sels organiques, désinfectants, polymères structurants, agents antimoussants, ingrédients encapsulés, agents d'ajustement du pH et additifs améliorant la sensation sur la peau ou nourrissants.

6. Utilisation d'un produit vaisselle à la main selon l'une des revendications 1 à 5 pour nettoyer des surfaces solides.

7. Procédé de nettoyage de surfaces dures, **caractérisé en ce qu'**un produit vaisselle à la main selon l'une des revendications 1 à 5 est utilisé dans au moins une étape du procédé.

8. Utilisation d'une combinaison d'enzymes comprenant au moins une protéase et au moins une amylase, dans laquelle
a) l'amylase présente une séquence d'acides aminés qui est identique à 100 % à la séquence d'acides aminés indiquée dans SEQ ID NO:1 sur toute sa longueur ; et
b) la au moins une protéase présente la séquence d'acides aminés selon SEQ **ID** NO:3, pour améliorer les performances de lavage d'un produit vaisselle à usage domestique.
